# FASCICULE DE BREVET EUROPEEN

(11) **EP 1 064 008 B1**
(45) Date de publication et mention de la délivrance du brevet: **08.01.2003**
(21) Numéro de dépôt: 99904917.4
(22) Date de dépôt: 19.02.1999
(51) Int. Cl.: A61K 35/78, A61P 17/00

(54) **UTILISATION D'AU MOINS UN EXTRAIT PROTEIQUE DE GRAINES DE PLANTES DU GENRE MORINGA ET COMPOSITION COSMETIQUE ET/OU PHARMACEUTIQUE CORRESPONDANTE**
VERWENDUNG VON MINDESTENS EINEM PROTEINEXTRAKT AUS PFLANZENSAMEN VON DER MORINGAGATTUNG UND ENTSPRECHENDE KOSMETISCHE ODER PHARMAZEUTISCHE ZUSAMMENSETZUNG
USE OF AT LEAST ONE PROTEIN EXTRACT OF THE MORINGA GENUS PLANT SEEDS AND CORRESPONDING COSMETIC AND/OR PHARMACOLOGICAL COMPOSITION

(30) Priorité: 24.03.1998 FR 9803787
(43) Date de publication de la demande: 03.01.2001
(73) Titulaire: Cognis France S.A., 31360 Saint Martory (FR)
(72) Inventeur: PAULY, Gilles, F-54000 Nancy (FR)
(74) Mandataire: Nuss, Pierre
(86) Numéro de dépôt international: FR9900384
(87) Numéro de publication internationale: WO99048512

(56) Documents cités:
- RICO MAGDA: "MORINGA:A HEALTH-GIVING, WATER-PURIFYING VEGETABLE" FOOD MARKETING & TECHNOLOGY, vol. 8, no. 6, décembre 1994, pages 10-11, XP002086663
- EILERT U. ET AL: 'The antibiotic principle of seeds of Moringa Oleifera and Moringa Stenopetala' PLANTA MEDICA vol. 42, Mai 1981, pages 55 - 61
- GASSENSCHMIDT U. ET AL: 'Isolation and characterization of a flocculating protein from Moringa Oleifera lam.' BIOCHIMICA ET BIOPHYSICA ACTA vol. 1243, 1995, pages 447 - 481

## Description

La présente invention concerne le domaine de la cosmétologie et de la dermatologie, plus particulièrement la cosmétique de soin pour la peau et les phanères, et a pour objet l'utilisation, pour des applications cosmétiques, dermatologique et/ou pharmaceutique, d'au moins un extrait protéique des graines d'une plante du genre Moringa, ainsi qu'une composition cosmétique et/ou pharmaceutique contenant au moins un tel extrait.

Le genre Moringa comprend quelques 14 espèces de plantes (dont notamment Moringa peregrina, M. aptera, M. concanensis, M. drouhardii, M. hildebrandtii, M. longituba), parmi lesquelles Moringa pterygosperma (synonyme Moringa oleifera) est la plus connue.

Il s'agit en l'occurrence d'un arbre à croissance rapide qui s'adapte très bien à des conditions variables, se développant partout dans les tropiques, en Asie, Afrique et Amérique du sud. Les fruits de 30 à 50 cm de long, pendent comme des baguettes de tambour d'où le nom anglais de "drumstick tree", et ses gousses vertes sont appréciées comme légume partout dans le monde. Il en résulte qu'on laisse rarement mûrir les graines en vue de la production d'huile.

Les différentes parties de l'arbre (feuilles, racines, écorce de racines, fleurs, graines) sont utilisées en médecine traditionnelle dans les pays où il pousse.

Les graines de Moringa sont caractérisées par la présence d'une huile dont la teneur varie entre 21 et 53 % selon l'espèce et la maturité des graines.

Pour l'espèce Moringa oleifera les teneurs mentionnées dans la littérature vont de 21 à 34 %.

La comparaison des huiles des graines de Moringa oleifera, M. peregrina, M. concanensis et M. drouhardii met en évidence une composition en acides gras très similaire, ces huiles présentant toutes un contenu très élevé en acide oléique (71 à 78 %) et en acides gras saturés.

L'acide béhénique (C₂₂H₄₄O₂) est l'acide gras saturé typique de l'huile de Moringa (teneur 2,6 à 4,7 %).

Du fait de son excellente stabilité à l'oxydation et de ses bonnes propriétés de fixation de parfums, l'huile de Moringa, également appelée huile de Behen ou de Ben, fut dans les civilisations antiques, l'huile la plus utilisée par les formulateurs d'onguents à usages cosmétiques et religieux.

Cette huile a été utilisée par les formulateurs en cosmétique jusqu'au siècle dernier et son utilisation a été "redécouverte" récemment.

En dehors de leur contenu en huile, les graines de Moringa ont récemment retenu l'attention des chercheurs car elles sont utilisées traditionnellement pour la clarification des eaux et possèdent donc un potentiel économique pour le traitement et la purification des eaux dans les pays en voie de développement.

Il a en outre été montré que les graines des six espèces de Moringa les plus fréquentes et cultivées contiennent des composés floculants.

Les composés responsables de cette activité ont été isolés et identifiés : il s'agit de composés de nature protéique (voir notamment les articles : "Isolation and caracterisation of a flocculating protein from Moringa oleifera lam", de GASSENSCHMIDT U., JANY K.D, TAUSCHE B et NIEBERGALL H.R, Biochimica et biophysica acta, 1243:477-481, 1995 - "Active agents and mechanism of coagulation of turbid waters using moringa oleifera", de NDABIGENGESERE A., SUBBA NARASIAH K. et TALBOT BG., Water research, 29, 2:703-710, 1995).

A partir de farine délipidée de graines de Moringa oleifera, ces protéines ont été extraites en milieu aqueux tamponné, puis isolées par chromatographie d'échange de cation.

Ces protéines floculantes sont éluées par un gradient NaCl et sont constituées par trois fractions actives appelées MO1, MO2, et MO3.

Une seconde étape chromatographique permet de séparer MO2 en trois nouvelles fractions actives appelées (MO2.1, MO2.2 et MO2.3).

En électrophorèse PAGE en conditions non dénaturantes, MO2.1, MO2.2 apparaissent comme homogènes alors que MO2.3 est constitué par plusieurs protéines.

Cependant en SDS-PAGE, deux bandes correspondant aux PM 6,5 kDa et 7 kDa sont observées pour les protéines MO2.1 et MO2.2 qui seraient donc des dimères.

L'isoélectrofocalisation montre que le point isoélectrique de ces protéines floculantes est de 10.

La composition en acides aminés de MO2.1 a été déterminée et montre que cette protéine contient 60 acides aminés avec un contenu élevé en glutamine (15 résidus), arginine (7 résidus) et proline (7 résidus), l'extrémité terminale étant bloquée par une fonction pyroglutamate.

Par ailleurs, le document "THE ANTIBIOTIC PRINCIPLE OF SEEDS OF MORINGA OLEIFERA AND MORINGA STENOPETALA", EILERT U. et AL., PLANTA MEDICA, vol. 42, mai 1981, pages 55-61, mentionne l'utilisation de compositions et de composés spécifiques, présentant une structure particulière et faisant état d'une activité anti-microbienne et antibiotique.

Les substances évoquées dans ce document consistent en des isothiocyanates pouvant éventuellement être obtenues à partir de graines de la plante Moringa Oleifera.

Les inventeurs de la présente invention ont découvert de façon inattendue et surprenante, que les extraits protéiques de graines de Moringa, connus pour leurs effets clarifiants sur les eaux turbides, présentaient également des propriétés nouvelles et originales dont les effets bénéfiques sur la peau et les phanères, associés à une très bonne tolérance, les rendent directement utilisables en cosmétique de soin et en pharmacologie, en particulier dans des applications dermatologiques.

Ainsi, le principal objet de la présente invention consiste en l'utilisation d'au moins une fraction protéique extraite des graines d'une plante du genre Moringa appartenant à la famille des Moringaceae, en tant que principe actif, seul ou en association avec au moins un autre principe actif, pour la préparation d'une composition cosmétique et/ou pharmaceutique à usage topique pour la peau et/ou les phanères.

Les propriétés nouvelles de ces extraits protéiques découlent directement de leur nature et propriétés particulières, notamment de leur point isoélectrique basique et de leur capacité floculante.

Il a notamment été constaté un effet adoucissant, des effets conditionneurs et hydratants en raison d'une substantivité importante et des effets chelateurs les rendant appropriées dans le cadre des actifs antipollution.

Conformément à un mode de réalisation préféré de l'invention, la ou les fraction(s) protéique(s)consiste(nt) en un ou des extraits de la plante Moringa oleifera, cette ou ces fraction(s) protéique(s) contenant sur la base de l'extrait sec, une teneur en protéines comprise entre 0,01 % et 100 % en poids préférentiellement d'environ 45 % en poids.

Avantageusement, la ou les fraction(s) protéique(s) est (sont) extraite(s) par l'eau ou une solution aqueuse, notamment des solutions salines à différents pH, le cas échéant au moyen d'un générateur à ultrasons.

Ainsi, la ou les fraction(s) protéique(s) est (sont) constituée(s) par un ou des extrait(s) aqueux, des extraits en milieux salins à différents pH, ou en milieu tamponné de graines entières ou décortiquées, partiellement ou totalement délipidées, par un concentré protéique, par des protéines purifiées ou par un mélange d'au moins deux des constituants précités et présente(nt) un point isoélectrique supérieur à 7, préférentiellement compris entre 8 et 12.

La ou les fraction(s) protéique(s) utilisée(s) en tant que principe actif sont préférentiellement obtenue(s) par :
- précipitation au point isoélectrique à un pH compris entre 8 et 12
- chromatographie d'échange d'ions.
- un procédé d'extraction choisi dans le groupe formé par la chromatographie d'affinité, la filtration sur gel, l'ultrafiltration, la précipitation à l'aide de solvants, de sels comme le sulfate d'ammonium ou autre ou encore la précipitation à l'aide de polymères organiques ou de variations de température.

En outre, il a été constaté que, dans le cas de la précipitation au point isoélectrique, l'obtention de la ou les fraction(s) protéique(s) était favorisée par une température inférieure à la température ambiante, notamment par une température autour de + 4° C.

A titre d'exemples illustratifs et non limitatifs, on décrira ci-après différents procédés d'obtention et de préparation d'extraits de graines et de fractions protéiques de graines de Moringa, utilisables dans le cadre de la présente invention.

### EXEMPLE 1 (Préparation de l'extrait 1)

Des amandes de Moringa oleifera obtenues après décorticage des graines et contenant 33,4 % (poids/poids) d'huile sont délipidées par deux extractions successives à reflux dans de l'hexane et, après filtration, la farine est séchée en étuve à 40° C et présente une teneur en huile résiduelle de 2,5 %.

Dans un réacteur on ajoute 200 g de farine délipidée à 2 litres d'eau distillée.

Après 10 minutes d'agitation, le pH est ajusté à 7,5 par addition de NaOH 4N et l'extraction est ensuite réalisée pendant une heure à température ambiante en maintenant le pH à 7,5.

L'insoluble est éliminé par centrifugation pendant 15 min. à 5000 g.

Le surnageant est recueilli puis filtré sur 0,45 µm : on obtient ainsi 1,77 litre de filtrat de couleur jaune, contenant 4,69 % d'extrait sec et présentant une concentration en protéines mesurée par la technique du Biuret de 21,54 g/l (soit une pureté protéique sur la base de l'extrait sec de 45,92 %).

L'extrait est déshydraté par atomisation et 65,72 grammes d'atomisat présentant une teneur en protéines estimée à 54,7 % (N x 6,25).

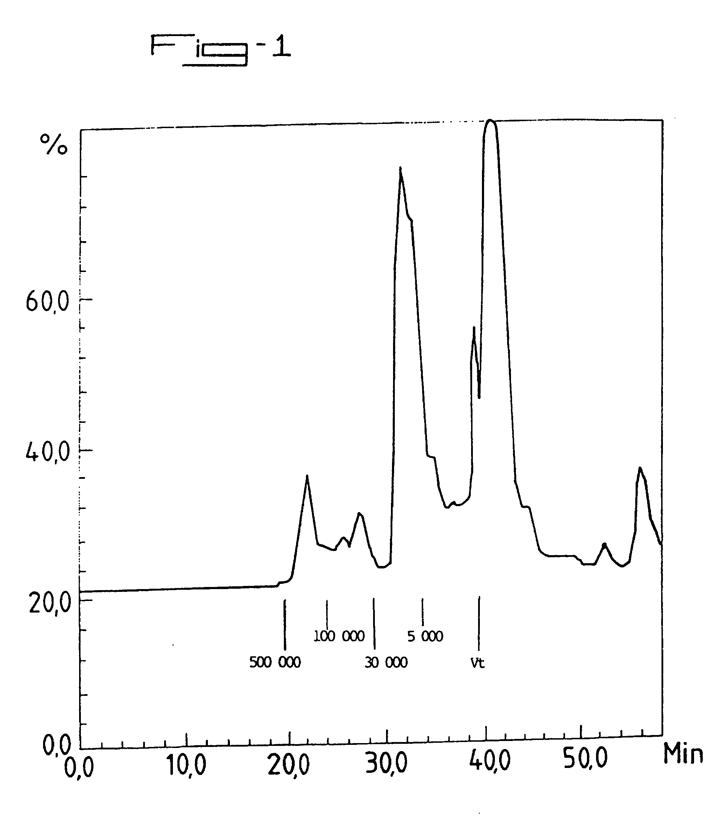

Si on tient compte des pics élués entre le volume exclu et le volume total de la colonne, le profil chromatographique résultant de l'analyse en perméation de gel sur colonne Superose 12HR de cet extrait (voir figure 1 des dessins annexés) met en évidence une fraction majeure qui représente 52 % de la surface et qui correspond à des poids moléculaires situés entre 7 800 et 11 000 Da. La présence d'épaulements dans ce pic confirme l'existence de plusieurs composés et la gamme de poids moléculaires est proche de celle trouvée dans la littérature pour les monomères (6 500 et 7 000 Da) et les dimères (13 000 Da) de protéines floculantes de Moringa.

### EXEMPLE 2 (Préparation de l'extrait 2)

On extrait 300 g de farine délipidée selon l'exemple 1 de manière à obtenir un extrait aqueux brut.

Le pH du filtrat (2,74 litres) est ajusté à 11,8 par addition progressive de NaOH 4N.

La précipitation débute vers pH 8,0 (trouble net de la solution) et après 30 minutes la solution est centrifugée pendant 15 min. à 5000 g.

Le précipité collant est recueilli (43,2 g humide) puis lavé deux fois par 500 ml d'eau distillée à pH 11,8.

Le précipité est ensuite dissout dans 270 ml d'eau distillée (soit 10 % du volume initial) et le pH de la solution est ajusté en continu à 4,5 par HCl 6N de façon à permettre la solubilisation du précipité (la dispersion est facilitée par l'emploi d'un appareil du type connu sous la désignation Turax).

Après 30 min d'agitation le mélange est centrifugé pendant 15 min. à 5000 g pour éliminer l'insoluble et le surnageant est filtré sur Büchner muni d'un filtre Whatman n° 41.

On obtient ainsi 260 ml de concentré protéique, jaune et limpide, qui est déshydraté par lyophilisation.

De cette manière, 11,5 grammes de lyophilisat sont obtenus avec une teneur pondérale en protéines de 90-95 %.

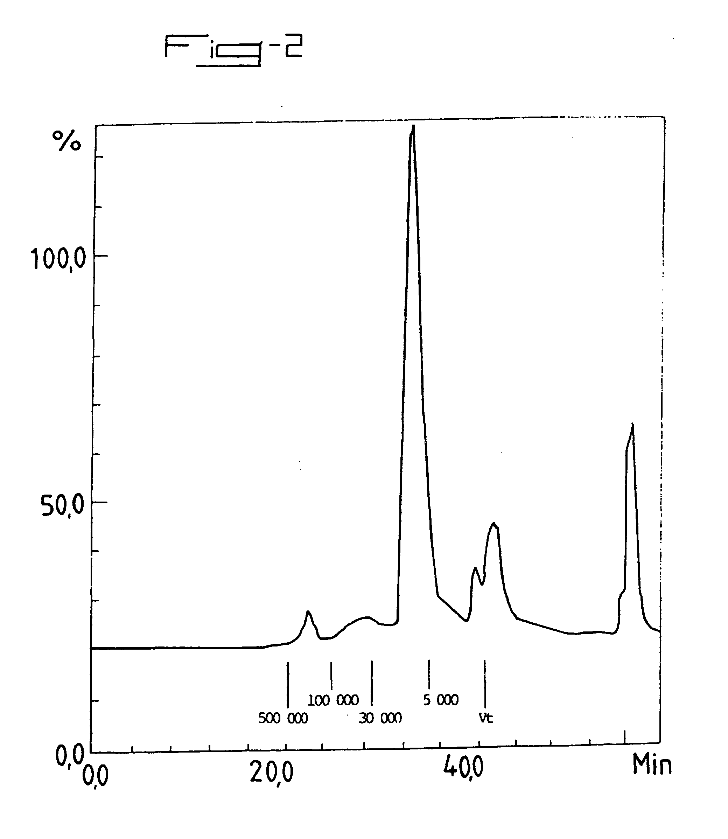

L'analyse en perméation de gel sur colonne Superose 12HR de cet extrait (voir figure 2 des dessins annexés) met en évidence une fraction majeure qui représente 70 % de la surface et qui correspond à des poids moléculaires d'environ 8 800 Da.

### EXEMPLE 3 (Préparation de l'extrait 3)

L'huile d'amandes obtenues par décorticage des graines de Moringa oleifera est extraite par pression sur une presse du type connu sous la désignation KOMET et les tourteaux obtenus sont broyés afin d'obtenir une farine homogène.

Un extrait brut est préparé à partir de 1,24 kg de tourteaux selon le mode opératoire décrit dans les exemples 1 et 2.

Les protéines sont précipitées à pH 11,8 selon l'exemple 2, mais une étape supplémentaire de décantation d'une nuit à + 4° C est introduite afin de permettre une meilleure précipitation des protéines.

Le précipité est traité dans les mêmes conditions que dans l'exemple 2 (le pH de la solution de reconstitution du précipité étant toutefois de 6 au lieu de 4,5).

Le concentré protéique ainsi obtenu (1,05 litre à 4,59 % d'extrait sec) est déshydraté par atomisation et 34,6 g d'atomisat sont recueillis, soit un rendement d'atomisation sur la base de l'extrait sec de 71,5 %.

La teneur en protéines sur la base du dosage de l'azote (N x 6,25) est supérieure à 90 % (environ 95 %).

### EXEMPLE 4 (Préparation de l'extrait 4)

Un extrait brut est préparé à partir de 150 g de tourteaux selon le mode opératoire décrit dans les exemples 1, 2 et 3.

Après filtration sur 0,45 µm, on obtient 1,35 litre de filtrat jaune limpide.

100 grammes de Carboxymethylcellulose (CM52, WHATMAN) sont mis à équilibrer pendant 30 minutes dans 500 ml d'eau distillée à pH 7,5.

Le mélange est filtré sur Büchner muni d'un filtre WHATMAN n° 42, puis la cellulose est recueillie et équilibrée de nouveau dans 500 ml d'eau à pH 7,5.

Après élimination du milieu aqueux par filtration, la cellulose est mise en contact, sous agitation pendant une heure à température ambiante, avec l'extrait aqueux de tourteaux d'amandes de Moringa oleifera.

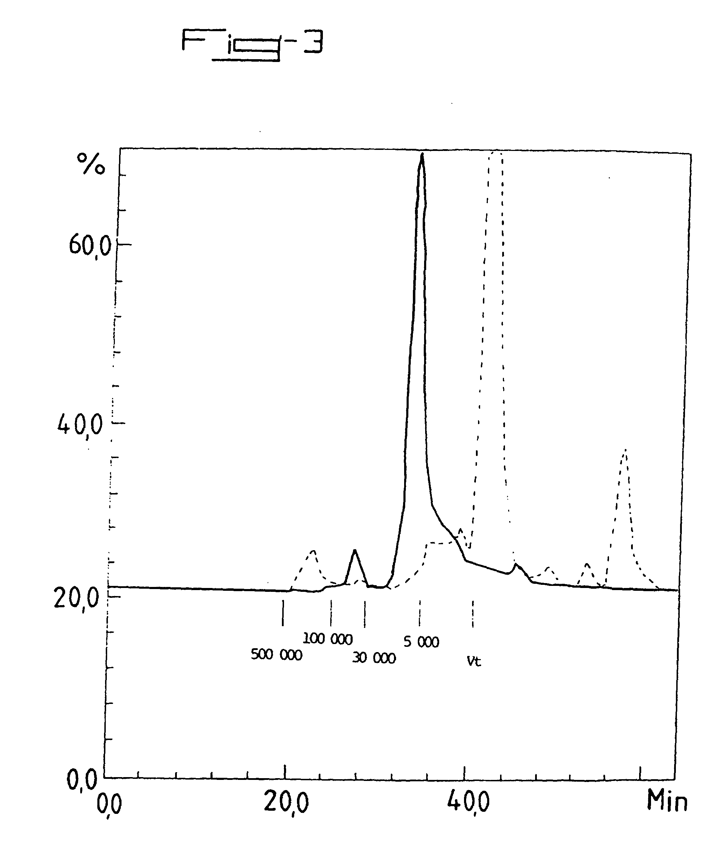

Les composés non adsorbés (fractions dont le profil chromatographique est représenté en trait interrompu sur la figure 3) sont éliminés par filtration sur Büchner et la cellulose "chargée" est ensuite lavée par deux fois avec un litre d'eau distillée à pH 7,5 puis filtrée sur Büchner.

La cellulose est ensuite mise en contact avec 120 ml d'une solution de NaCl 60 g/l pH 7,5 pendant 30 minutes.

Les protéines éluées en milieu NaCl sont récupérées par filtration sur Büchner (le profil chromatographique des protéines adsorbées sur CM 52 et éluées en milieu NaCl 60 g/l est représenté en trait plein sur la figure 3).

On obtient ainsi 110 ml de filtrat avec une teneur d'extrait sec de 9,63 % et avec une concentration en protéines de 64,6 g/l (soit une pureté protéique sur la base de l'extrait sec de 67 %).

L'analyse en perméation de gel sur colonne Superose 12HR de cet extrait (voir figure 3) met en évidence une fraction majeure qui représente 70 % de la surface et qui correspond à des poids moléculaires d'environ 7 100 Da.

La solution peut être dessalée par dialyse, ou par ultrafiltration et déshydratée par lyophilisation, atomisation ou tout autre moyen approprié.

Les activités et avantages des produits préparés selon l'invention apparaîtront clairement à la lumière de la description explicative des tests réalisés par les inventeurs et donnés ci-après simplement à titre d'illustration non limitative.

L'effet substantif des fractions protéiques de graines de Moringa préparées selon les exemples précédents (extraits 1, 3 et 4) a été évalué par le test de désorption sur cheveux humains naturels non endommagés et cheveux endommagés par permanentage.

Le protocole d'évaluation d'absorption d'un agent substantif sur la kératine des cheveux est basé sur l'analyse de la substance désorbée dans des conditions spécifiques.

La protéine substantive de nature peptidique a été extraite à partir des cheveux dans deux conditions différentes :
- température élevée (50° C, 1 heure),
- force ionique élevée (0,5 M NaCl, 16 heures),
et dosée dans les liquides d'extraction après réaction avec la fluorescamine (réaction avec les amines primaires) par méthode spectrofluorimétrique (Teglia et al, 1992). La réaction avec la fluorescamine a été effectuée dans le tampon borate à pH 8 pour l'extrait 1 et à pH 6 pour les extraits 3 et 4.

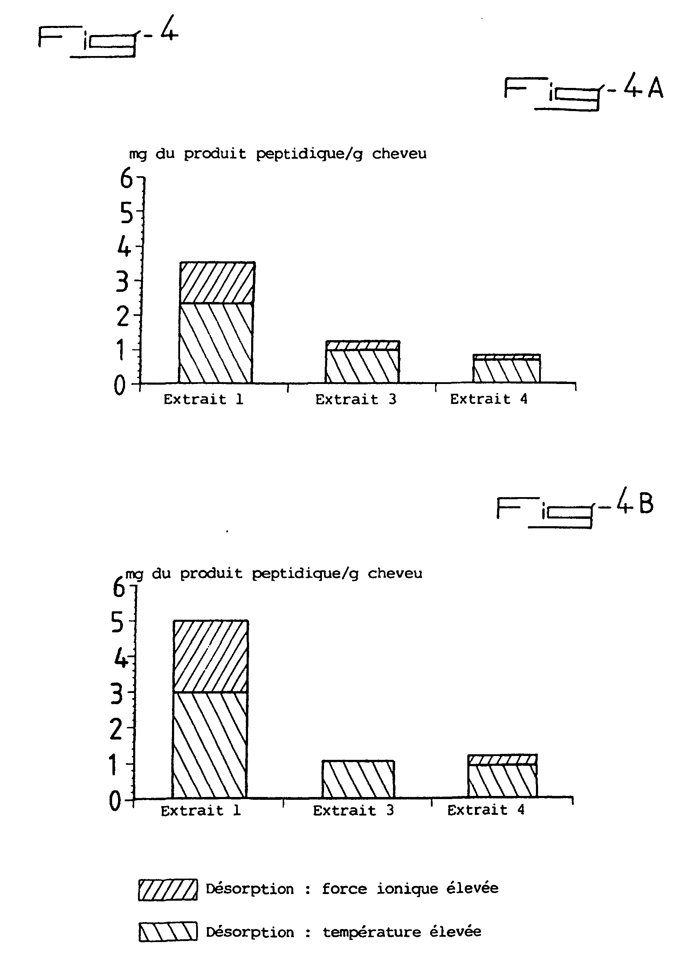

Les résultats sont résumés dans le tableau I ci-après et sur les figures 4A et 4B annexés qui illustrent, respectivement pour des cheveux témoin non endommagés (Fig. 4A) et pour des cheveux endommagés par permanentage (Fig. 4B), l'effet substantif des extraits 1, 3 et 4 appliqués sur des mèches de cheveux humains à la concentration de 2 % (évaluation par désorption).

**Tableau I**

| Extraits | Cheveux naturels non endommagés | Cheveux endommagées par permanentage |
|---|---|---|
| 1 | 3,53 mg/g cheveux | 4,81 mg/g cheveux |
| 3 | 1,24 mg/g cheveux | 1,02 mg/g cheveux |
| 4 | 0,84 mg/g cheveux | 1,21 mg/g cheveux |

L'effet substantif sur couche cornée des fractions protéiques de graines de Moringa a été évalué sur le modèle d'hydratation de la couche cornée in vitro (Obata et Tagami, 1990).

Le protocole d'évaluation a consisté à traiter des carrés de couche cornée isolée de peau humaine, soit par des solutions à 2 % de protéine substantive (extraits 1, 3 et 4), soit par l'eau distillée comme témoin.

Après rinçage standard et séchage, la couche cornée est montée sur un modèle d'évaluation d'hydratation cutanée in vitro par mesure de la conductibilité diélectrique.

Après mesure de la conductibilité diélectrique de départ en condition d'humidité contrôlée HR = 44 %, la couche cornée a été humidifiée de façon standard et des mesures de conductibilité ont été effectuées 1, 2, 4, 6 et 24 heures après l'application de l'eau distillée.

La couche cornée en présence d'un produit substantif est hydratée plus longtemps (= meilleure rétention d'eau).

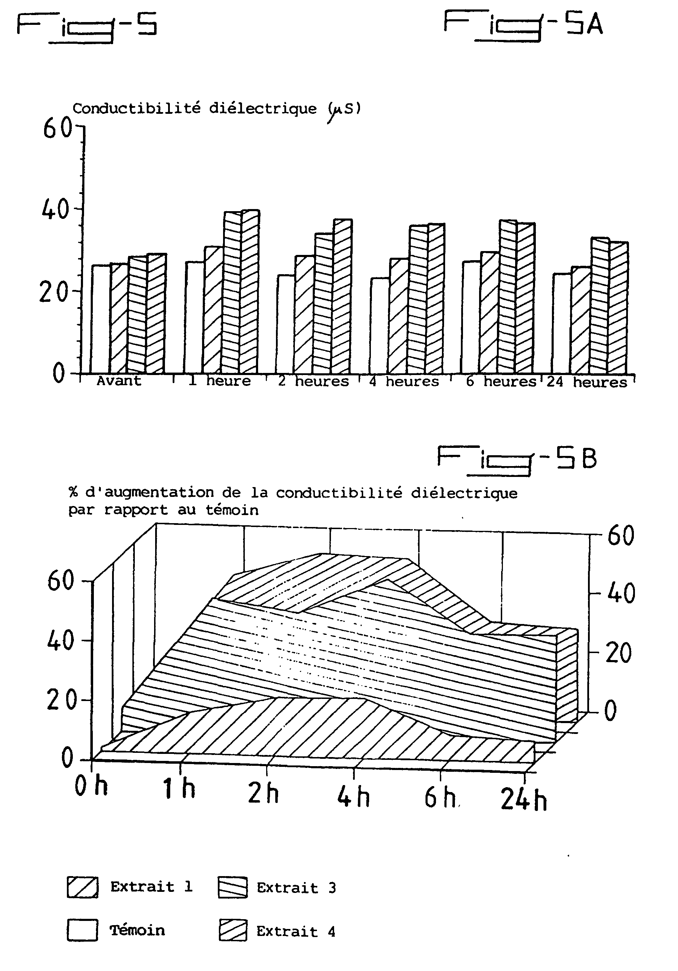

Les résultats relatifs aux tests de substantivité des extraits 1, 3 et 4 sur la couche cornée évaluée par mesures d'hydratation cutanée in vitro sont résumés par les figures 5A et 5B (Moyenne de 10 essais / +/- SEM / ANOVA à 1 facteur / Test a posteriori Fischer).

On remarque que les extraits 3 et 4 ont accentué et prolongé l'effet hydratant de la couche cornée : l'augmentation de la conductibilité diélectrique de 50 % a été significative jusqu'à 4 heures après application, avec un effet hydratant observable jusqu'à 24 heures après application.

L'effet substantif des fractions protéiques de Moringa (extraits 1, 3 et 4) a été vérifié in vivo chez l'homme par l'étude de la propriété d'hydrorétention d'une solution aqueuse dosée à 1,5 % d'extrait et à 1,5 % de polyvinyl pyrrolidone commercialisé par la société BASF sous la dénomination Kollidon 30 (produit filmogène).

Il a été procédé sur cinq zones cutanées de la face antéro-interne d'un avant-bras, à la mesure par capacitance électrique de l'hydratation apportée par une compresse imbibée d'eau purifiée, toutes les 30 secondes pendant 2 minutes. Une zone était non traitée, les trois suivantes étaient traitées au préalable par 4 µl/cm² d'une solution aqueuse dosée à 1,5 % d'extrait 1,3 ou 4 et à 1,5 % de Kollidon 30 et la dernière par 4 µl/cm² d'une solution aqueuse dosée à 1,5 % de Kollidon 30 seul.

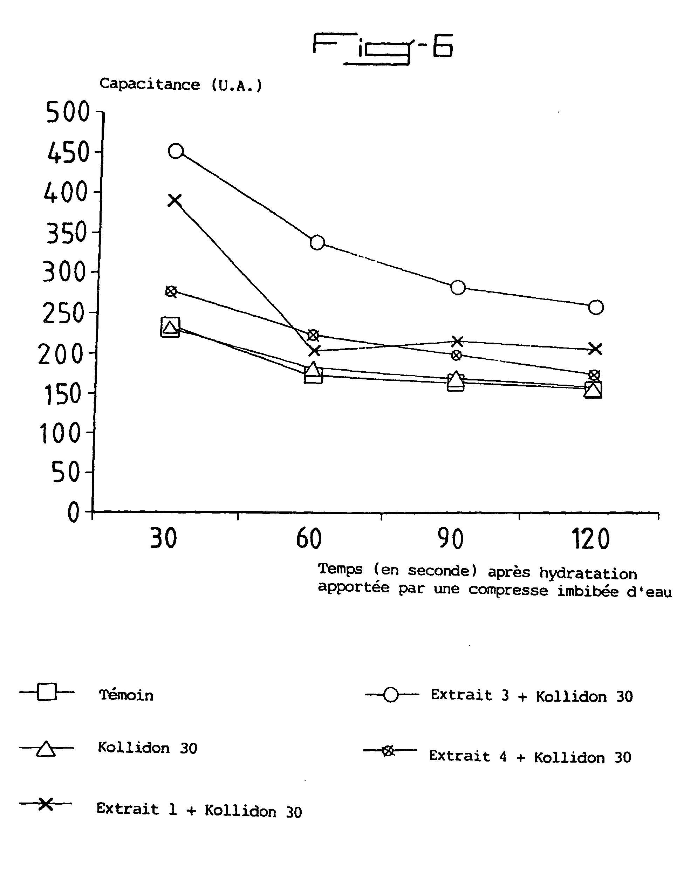

Les résultats présentés sur la figure 6 des dessins annexés (illustrant l'hydrorétention sur un sujet) montrent que l'hydratation de la peau est plus importante quand la zone cutanée est prétraitée par le mélange extrait + Kollidon 30 comparativement à une zone témoin non traitée ou contenant le filmogène seul.

Les solutions dosées à 1,5 % d'extrait + 1,5 % de Kollidon 30 possèdent donc une activité hydrorétentrice par effet substantif.

Déjà connu pour leurs effets clarifiant sur les eaux turbides, les protéines de Moringa ont également démontré, de façon surprenante et inattendue, un effet anti-pollution sur la peau humaine par captation de particules comme le charbon végétal.

Cette propriété clarifiante des fractions protéiques de graines de Moringa a été vérifiée in vitro, en introduisant dans des tubes à essai, 0,75 ml d'une solution aqueuse de l'extrait 1 avec 3 ml de suspension aqueuse de charbon végétal officinal à 0,5 %, comparativement à 0,75 ml d'eau distillée mis également en contact avec 3 ml de la même suspension aqueuse de charbon.

Il a été observé, après une heure de contact, que dans le tube contenant l'extrait 1, la suspension noire de charbon d'origine avait laissé la place à un précipité noir au fond du tube avec en surnageant une eau 'parfaitement limpide, alors que dans le tube sans l'extrait, la suspension aqueuse de charbon noire avait très peu évolué.

Il a été constaté par ailleurs que, de façon avantageuse, l'addition d'un composé filmogène comme le Kollidon 30 (marque déposée - Polyvinylpyrrolidone), accélérait encore le processus de clarification.

Un test in vivo chez l'homme spécialement mis au point, a permis d'observer qu'une suspension aqueuse de charbon végétal officinal polluante appliquée sur la peau, provoquait une salissure importante en surface avec pénétration des particules dans les niveaux superficiels de la couche cornée.

L'efficacité de l'extrait 1 comme dépolluant cutané contre cet effet salissant du charbon et contre la pénétration de particules polluantes dans la couche cornée, a également été évaluée expérimentalement.

Le test réalisé en conditions standardisées a consisté à appliquer sur la face antéro-interne de l'avant-bras un mélange d'extrait de Moringa et de Kollidon, dosés chacun à 1,5 % dans l'eau, à raison de 4 mg/cm². Ce traitement a été suivi par une pollution volontaire de la peau avec la même suspension aqueuse de charbon dosée à 0,5 %. La peau a ensuite été rincée à l'eau distillée sans frotter, en réaspirant l'eau de rinçage.

Puis un "stripping" ou gommage (support de pelliculage) a été appliqué sur la peau, puis retiré.

En microscopie au grossissement x 100, on a observé, d'une part, le stripping témoignant de l'état de la peau en surface et, d'autre part, le microrelief cutané avec les premiers niveaux de la couche cornée, après stripping ou gommage.

Parallèlement, la même pollution a été appliquée sur une zone cutanée sans traitement préalable.

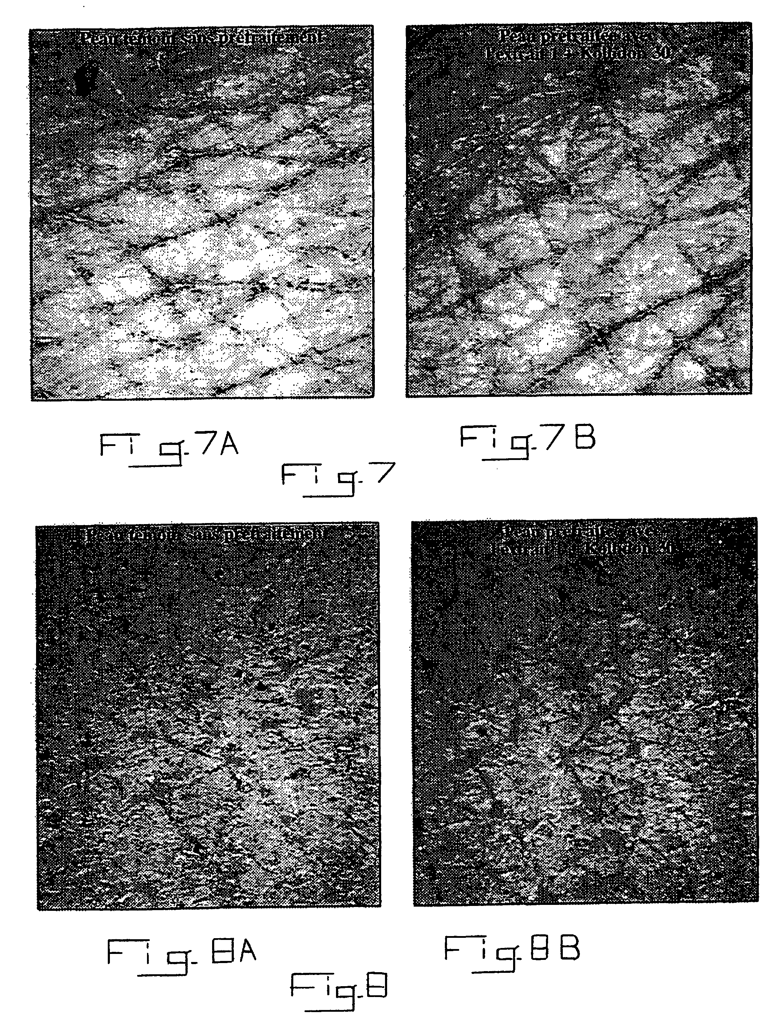

Les images de la peau sous grossissement x 100 après stripping ou gommage ont permis de mettre en évidence une très importante diminution de la pénétration des particules de charbon dans la couche cornée de la peau prétraitée par le mélange d'extrait de Moringa et de Kollidon (Fig. 7B) comparativement à la peau témoin (Fig. 7A) pour laquelle la pénétration était beaucoup plus importante.

Les images en microscopie des strippings effectués sur la peau prétraitée par le mélange extrait + Kollidon (Fig. 8B) ont montré une importante diminution de la quantité de particules de charbon, prouvant que le même mélange d'extrait et de Kollidon avait permis au cours de l'opération de rinçage, un nettoyage de la peau beaucoup plus efficace. En effet, par comparaison avec le stripping de la peau non traitée au préalable par l'extrait (Fig. 8A), les nombreuses particules de charbon qui n'avaient pas été éliminées par l'opération de rinçage, étaient restées collées sur le stripping.

L'extrait de Moringa exerce donc une activité anti-pollution de deux façons distinctes et complémentaires :
- il capte les particules de charbon et, allié à un filmogène, il empêche ces dernières de pénétrer dans la couche cornée,
- en retenant les particules de charbon, il favorise leur élimination au cours d'une simple opération de rinçage.

Les extraits selon l'invention précités, peuvent être utilisés, non seulement pour des applications de soins et d'hygiène de la peau (produits pour le visage et le corps, produits de jour ou de nuit, produits solaires, produit hygiène anti-rides, produits antipollution), mais également dans le domaine des soins et de l'hygiène capillaires, (lotion ou shampooing, crèmes, mousses, produits protecteurs, réparateurs, adoucissants et photoprotecteurs ou encore produits pour permanente et de coloration), des produits pour les ongles (crèmes, lotions, vernis hydratants, filmogènes, protecteurs et réparateurs) et enfin des produits pour les lèvres (bâtons applicateurs, rouge à lèvres, baume, hydratant, filmogène).

Ainsi, la présente invention a également pour objet une composition cosmétique et/ou pharmaceutique, notamment à usage topique pour la peau ou les phanères, caractérisée en ce qu'elle contient, à titre de principe actif, unique ou associé à au moins un autre principe actif, une ou plusieurs fraction(s) protéique(s) telle(s) que décrite(s) ci-dessus, avec une teneur pondérale comprise entre 0,01 % et 80 %, préférentiellement d'environ 2 %.

Les fractions protéiques obtenues selon la présente invention peuvent être utilisées soit sous forme native (protéines natives), sans modification de structure, soit sous une forme modifiée ou fonctionnalisée par l'un quelconque des traitements suivants
- polymérisation des protéines natives extraites de la plante du genre Moringa,
- fermentation par des cellules microbiennes ou végétales,
- hydrolyse chimique des protéines natives extraites de la plante du genre Moringa,
- hydrolyse enzymatique des protéines natives par des protéases d'origine animale végétales, microbienne ou fongique,
- la fonctionnalisation chimique ou enzymatique,
- modification chimique par greffage de molécules ou de composés tels que, par exemple, des oses, des osides, des lipides ou autre.

Elles peuvent également être incorporées dans ou associées à tout vecteur cosmétique adéquat comme, par exemple, les agents filmogènes, les liposomes, les cyclodextrines, les micelles, les chylomicrons, les macro-, micro- et nano particules ainsi que macro-, micro- et nano capsules, ou encore être absorbées ou greffées sur des polymères organiques ou des supports minéraux.

Par conséquent, il a été constaté et démontré ci-dessus que les fractions protéiques extraites présentent notamment des activités substantive et hydratante de la peau, des lèvres et des phanères, en particulier de l'épiderme, et notamment de la couche cornée et des annexes cutanées, à savoir peau, cheveux et ongles, ainsi que des effets conditionneurs physiologiques de la peau, des lèvres et des phanères (ongles et cheveux), restructurant, réparateur et hydratant de la peau et des phanères (ongles et cheveux), des effets antirides et des effets antipollution (peau, lèvres, cheveux).

A titre d'exemples non limitatifs de réalisations pratiques de composition selon l'invention, on décrira ci-après différents produits ou préparations cosmétiques comprenant au moins un extrait protéique de graines de la plante Moringa oleifera.

### Exemple 1

Un produit cosmétique sous forme de lait hydratant et réparateur pourra, par exemple, présenter une composition pondérale, constituée à partir des phases aqueuses et grasses suivantes, telle qu'indiquée ci-après.

| | | |
|---|---|---|
| Phase grasse | Succinate d'Isostéaryle et de Diglycéryle | 3,00 |
| | Huile de paraffine | 15,00 |
| | Quaternium-18 Hectorite | 0,50 |
| | Poly(PEG-22/Dodécyle Glycol) | 1,00 |
| Phase aqueuse | Sulfate de magnésium | 0,80 |
| | Butylène glycol | 4,00 |
| | Extr. protéinique de Moringa oleifera (selon Ex. 1) et | 1,00 |
| | Eau distillée | 9,00 |
| | Elestab 4112 (Laboratoires Sérobiologiques) | 0,35 |
| | Parfum | 0,30 |
| | Eau distillée qsp | 100,00 |

Le procédé de préparation du lait hydratant et réparateur précité consiste essentiellement à porter la phase grasse à 80° C, à porter l'eau de la phase aqueuse également à 80° C et à y dissoudre le préservateur, (Elestab 4112), puis à verser la phase aqueuse dans la phase grasse sous agitation turbine et à refroidir progressivement sous agitation, à y ajouter ensuite, vers 50° C, la solution mère aqueuse d'extrait protéique de Moringa, puis le parfum et, enfin, à poursuivre l'agitation jusqu'à refroidissement complet.

### Exemple 2

Un produit cosmétique sous forme de crème réparatrice antirides pourra, par exemple, présenter une composition pondérale, constituée à partir des phases aqueuse et grasse suivantes, telle qu'indiquée ci-après.

| | | |
|---|---|---|
| Phase grasse | Ceteareth 25 | 2,00 |
| | Ceteareth 6 (and) Stearyl Alcool stéarylique | 1,00 |
| | Alcool cétylique | 4,00 |
| | Stéarate de glycérol | 4,00 |
| | Pétrolatum | 5,00 |
| | Triglycérides capryliques / capriques | 5,00 |
| Phase aqueuse | Glycérine | 10,00 |
| | Protéines de Moringa Oleifera (préparée selon ex. 2) et | 1,50 |
| | Eau distillée | 8,50 |
| | Préservateur Elestab 4112 (Laboratoires Sérobiologiques) | 0,40 |
| | Parfum | 0,30 |
| | Eau distillée qsp | 100 |

Le procédé de préparation de la crème réparatrice antirides précitée consiste essentiellement à porter la phase grasse à 80° C, à porter la phase aqueuse également à 80° C et à y dissoudre l'Elestab 4112, à préparer séparément la solution mère d'extrait protéique de Moringa Oleifera, à verser la phase grasse dans la phase aqueuse sous agitation turbine, puis, aux environs de 50° C, à y introduire la solution mère d'extrait de Moringa et enfin à poursuivre l'agitation jusqu'au refroidissement.

### Exemple 3

Un produit cosmétique sous forme de crème de jour hydratante et antipollution pourra, par exemple, présenter une composition pondérale, constituée à partir des phases aqueuse et grasse suivantes, telle qu'indiquée ci-après.

| | | |
|---|---|---|
| Phase grasse | Stéarate de glycérol | 14,00 |
| | Octyldodécanol | 6,00 |
| | Adipate dibutylique | 6,00 |
| | Ceteareth 12 | 1,50 |
| | Ceteareth 20 | 1,50 |
| Phase aqueuse | PVP | 0,50 |
| | Glycérine | 4,00 |
| | Elestab 388 (Laboratoires Sérobiologiques) | 2,00 |
| | Extrait protéique de Moringa (Ext. n° 2) et | 1,00 |
| | Eau distillée | 9,00 |
| | Parfum | 0,20 |
| | Eau distillée qsp | 100 |

Le procédé de préparation de la crème de jour hydratante et antipollution précitée consiste essentiellement à porter la phase grasse à 80° C, à porter la phase aqueuse également à 80° C et à y dissoudre Elestab 388 et PVP, à verser la phase grasse dans la phase aqueuse sous agitation turbine à 80° C, puis, à refroidir progressivement sous agitation, à y introduire ensuite, aux environs de 50° C, la dispersion mère de protéines de Moringa et enfin à poursuivre l'agitation jusqu'au refroidissement.

### Exemple 4

Un produit cosmétique sous forme de lotion capillaire non rincée réparatrice antipollution et biofilmogène pourra, par exemple, présenter une composition pondérale telle qu'indiquée ci-après.

| | |
|---|---|
| Protéines de Moringa préparées selon exemple 4 | 0,50 |
| Eau distillée | 9,50 |
| Hydroxyéthylcellulose | 0,50 |
| Elestab 305 (Laboratoires Sérobiologiques) | 0,50 |
| Parfum | 0,10 |
| Cremophor RH40 | 0,30 |
| Eau distillée qsp | 100,00 |

Le procédé de préparation de la lotion capillaire non rincée réparatrice antipollution et biofilmogène consiste essentiellement à dissoudre Elestab 505 et hydroxyéthylcellulose dans l'eau chauffée aux environs de 50° C, à y disperser le parfum et Cremophar RH40, puis à ramener le mélange à température ambiante, à y dissoudre ensuite les protéines de Moringa et enfin à réaliser un filtrage.

Bien entendu, l'invention n'est pas limitée aux modes de réalisation décrits. Des modifications restent possibles, notamment du point de vue de la constitution des divers éléments ou par substitution d'équivalents techniques, sans sortir pour autant du domaine de protection de l'invention.

## Revendications

1. Utilisation d'au moins une fraction protéique extraite des graines d'une plante du genre Moringa appartenant à la famille des Moringaceae, en tant que principe actif, seul ou en association avec au moins un autre principe actif, pour la préparation d'une composition cosmétique et/ou pharmaceutique à usage topique pour la peau, les lèvres et/ou les phanères.

2. Utilisation selon la revendication 1, **caractérisée en ce que** la ou les fraction(s) protéique(s)consiste(nt) en un ou des extraits de la plante Moringa oleifera.

3. Utilisation selon les revendications 1 et 2, **caractérisée en ce que** la ou les fraction(s) protéique(s) est (sont) extraite(s) par l'eau ou une solution aqueuse, notamment des solutions salines à différents pH, le cas échéant au moyen d'un générateur à ultrasons.

4. Utilisation selon l'une quelconque des revendications 1 à 3, **caractérisée en ce que** la ou les fraction(s) protéique(s) est (sont) constituée(s) par un ou des extrait(s) aqueux ou en milieu tamponné de graines entières ou décortiquées partiellement ou totalement délipidées, par un concentré protéique, par des protéines purifiées ou par un mélange d'au moins deux des constituants précités.

5. Utilisation selon l'une quelconque des revendications 1 à 4, **caractérisée en ce que** la ou les fraction(s) protéique(s) contien(en)t, sur la base de l'extrait sec, une teneur en protéines comprise entre 0,01 % et 100 % en poids préférentiellement d'environ 45 % en poids.

6. Utilisation selon l'une quelconque des revendications 1 à 5, **caractérisée en ce que** les fraction(s) protéique(s) comporte(nt) au moins un composé protéique dont le point isoélectrique est supérieur à 7, préférentiellement compris entre 8 et 12.

7. Utilisation selon l'une quelconque des revendications 1 à 6, **caractérisée en ce que** la ou les fraction(s) protéique(s) est (sont) obtenue(s) par précipitation au point isoélectrique à un pH compris entre 8 et 12.

8. Utilisation selon l'une quelconque des revendications 1 à 6, **caractérisée en ce que** la ou les fraction(s) protéique(s) est (sont) obtenue(s) par chromatographie d'échange d'ions.

9. Utilisation selon l'une quelconque des revendications 1 à 6, **caractérisée en ce que** la ou les fraction(s) protéique(s) est (sont) obtenue(s) par un procédé de préparation choisi dans le groupe formé par la chromatographie d'affinité, la filtration sur gel, l'ultrafiltration, la précipitation à l'aide de solvants, de sels comme le sulfate d'ammonium ou autre ou encore la précipitation à l'aide de polymères organiques ou de variations de température.

10. Utilisation selon l'une quelconque des revendications 1 à 9, **caractérisée en ce que** la ou les fraction(s) protéique(s) consiste(nt) en des protéines natives.

11. Utilisation selon l'une quelconque des revendications 1 à 9, **caractérisée en ce que** la ou les fraction(s) protéique(s) consiste(nt) en un hydrolysat chimique ou enzymatique préparé à partir des protéines natives.

12. Utilisation selon l'une quelconque des revendications 1 à 9, **caractérisée en ce que** la ou les fraction(s) protéique(s) est (sont) obtenue(s) par polymérisation des protéines natives extraites de la plante du genre Moringa ou fermentation par des cellules microbiennes ou végétales.

13. Utilisation selon l'une quelconque des revendications 1 à 9, **caractérisée en ce que** la ou les fraction(s) protéique(s) sont chimiquement modifiées par greffage de composés tels que, par exemple, des oses, des osides, des lipides ou analogues.

14. Utilisation selon l'une quelconque des revendications 1 à 13, **caractérisée en ce que** les fractions protéiques sont incorporées ou associées à un vecteur cosmétique adéquat tels que, par exemple, des agents filmogènes, des liposomes, des cyclodextrines, des micelles, des chylomicrons, des macro-, micro- ou nano-particules ou encore des macro-, micro- ou nano-capsules.

15. Utilisation selon l'une quelconque des revendications 1 à 13, **caractérisée en ce que** les fractions protéiques sont absorbées ou greffées sur des polymères organiques ou des supports minéraux.

16. Utilisation selon l'une quelconque des revendications 1 à 13, **caractérisée en ce que** les fractions protéiques extraites présentent des activités substantive et hydratante de la peau, des lèvres et des phanères, en particulier de l'épiderme, et notamment de la couche cornée et des annexes cutanées, à savoir peau, cheveux et ongles.

17. Utilisation selon l'une quelconque des revendications 1 à 13, **caractérisée en ce que** les fractions protéiques extraites présentent des effets conditionneurs physiologiques de la peau, des lèvres et des phanères restructurant, réparateur et hydratant de la peau, des lèvres et des phanères ainsi que des, effets antirides et effets antipollution.

18. Composition cosmétique et/ou pharmaceutique, notamment à usage topique pour la peau ou les phanères, **caractérisée en ce qu'**elle contient, à titre de principe actif, unique ou associé à au moins un autre principe actif, une ou plusieurs fraction(s) protéique(s) selon l'une quelconque des revendications 1 à 17, avec une teneur pondérale comprise entre 0,01 % et 80 %, préférentiellement d'environ 2 %.

## Patentansprüche

1. Verwendung mindestens einer aus Samen einer Pflanze der Gattung Moringa, die zur Familie der Moringaceae gehört, extrahierten Proteinfraktion als Wirkstoff allein oder zusammen mit mindestens einem weiteren Wirkstoff zur Herstellung einer Kosmetik- und/oder Arzneimittelzusammensetzung zur topischen Verwendung für die Haut, die Lippen und/oder die Epithelanhanggebilde.

2. Verwendung nach Anspruch 1, **dadurch gekennzeichnet, daß** die Proteinfraktion(en) aus einem oder mehreren Extrakten der Pflanze Moringa oleifera besteht bzw. bestehen.

3. Verwendung nach den Ansprüchen 1 und 2, **dadurch gekennzeichnet, daß** die Proteinfraktion(en) mit Wasser oder einer wäßrigen Lösung, insbesondere Salzlösungen mit unterschiedlichen pH-Werten, gegebenenfalls mittels eines Ultraschallgeräts, extrahiert wird bzw. werden.

4. Verwendung nach einem der Ansprüche 1 bis 3, **dadurch gekennzeichnet, daß** die Proteinfraktion(en) aus einem oder mehreren wäßrigen Extrakten oder aus (einem) mit einem gepufferten Medium gewonnenen Extrakt(en) aus ganzen Samen oder Samen, bei denen die Samenschalen entfernt wurden, die teilweise oder ganz entfettet wurden, aus einem Proteinkonzentrat, aus gereinigten Proteinen oder aus einer Mischung aus mindestens zwei der obengenannten Bestandteile besteht bzw. bestehen.

5. Verwendung nach einem der Ansprüche 1 bis 4, **dadurch gekennzeichnet, daß** die Proteinfraktion(en) in bezug auf den Trockenextrakt einen Proteingehalt von 0,01 Gew.-% bis 100 Gew.-%, vorzugsweise ungefähr 45 Gew.-%, aufweist bzw. aufweisen.

6. Verwendung nach einem der Ansprüche 1 bis 5, **dadurch gekennzeichnet, daß** die Proteinfraktion(en) mindestens eine Proteinverbindung enthält bzw. enthalten, deren isoelektrischer Punkt über 7, vorzugsweise zwischen 8 und 12, liegt.

7. Verwendung nach einem der Ansprüche 1 bis 6, **dadurch gekennzeichnet, daß** die Proteinfraktion(en) durch Fällung beim isoelektrischen Punkt bei einem pH zwischen 8 und 12 erhalten wird bzw. werden.

8. Verwendung nach einem der Ansprüche 1 bis 6, **dadurch gekennzeichnet, daß** die Proteinfraktion(en) mittels Ionenaustauschchromatographie erhalten wird bzw. werden.

9. Verwendung nach einem der Ansprüche 1 bis 6, **dadurch gekennzeichnet, daß** die Proteinfraktion(en) durch ein Herstellungsverfahren aus der Gruppe Affinitätschromatographie, Gelfiltration, Ultrafiltration, Fällung mit Lösungsmitteln, mit Salzen wie Ammoniumsulfat o.a. oder auch Fällung mit Hilfe von organischen Polymeren oder Temperaturvariationen erhalten wird bzw. werden.

10. Verwendung nach einem der Ansprüche 1 bis 9, **dadurch gekennzeichnet, daß** die Proteinfraktion(en) aus nativen Proteinen besteht bzw. bestehen.

11. Verwendung nach einem der Ansprüche 1 bis 9, **dadurch gekennzeichnet, daß** die Proteinfraktion(en) aus einem aus nativen Proteinen hergestellten chemischen oder enzymatischen Hydrolysat besteht bzw. bestehen.

12. Verwendung nach einem der Ansprüche 1 bis 9, **dadurch gekennzeichnet, daß** die Proteinfraktion(en) durch Polymerisation der nativen, aus der Pflanze der Gattung Moringa extrahierten Proteine oder durch Fermentation mit Mikroorganismen- oder Pflanzenzellen erhalten wird bzw. werden.

13. Verwendung nach einem der Ansprüche 1 bis 9, **dadurch gekennzeichnet, daß** die Proteinfraktion(en) mittels Pfropfung von Verbindungen wie zum Beispiel Osen, Osiden, Lipiden oder Analogen chemisch modifiziert wird bzw. werden.

14. Verwendung nach einem der Ansprüche 1 bis 13, **dadurch gekennzeichnet, daß** die Proteinfraktionen in ein geeignetes kosmetisches Übertragungsmittel wie zum Beispiel Filmbildner, Liposome, Cyclodextrine, Micellen, Chylomikrone, Makro-, Mikro- oder Nanopartikel oder Makro-, Mikro- oder Nanokapseln eingearbeitet oder mit diesen kombiniert werden.

15. Verwendung nach einem der Ansprüche 1 bis 13, **dadurch gekennzeichnet, daß** die Proteinfraktionen auf organische Polymere oder anorganische Träger absorbiert oder auf diese aufgepfropft werden.

16. Verwendung nach einem der Ansprüche 1 bis 13, **dadurch gekennzeichnet, daß** die extrahierten Proteinfraktionen eine direkte, feuchtigkeitsspendende Wirkung auf die Haut, die Lippen und die Epithelanhanggebilde, besonders die Epidermis, insbesondere das Stratum corneum und die Hautanhanggebilde, nämlich Haut, Haare und Nägel, haben.

17. Verwendung nach einem der Ansprüche 1 bis 13, **dadurch gekennzeichnet, daß** die extrahierten Proteinfraktionen physiologische Konditionierungswirkungen auf die Haut, die Lippen und die Epithelanhanggebilde ausüben und dabei die Haut, die Lippen und die Epithelanhanggebilde restrukturieren, wiederaufbauen und ihnen Feuchtigkeit spenden, und außerdem Wirkungen gegen Falten und Wirkungen gegen die Umweltverschmutzung aufweisen.

18. Kosmetik- und/oder Arzneimittelzusammensetzung insbesondere zur topischen Verwendung für die Haut oder die Epithelanhanggebilde, **dadurch gekennzeichnet, daß** sie als Wirkstoff allein oder zusammen mit mindestens einem weiteren Wirkstoff eine oder mehrere Proteinfraktion(en) nach einem der Ansprüche 1 bis 17 mit einem Gehalt von 0,01 Gew.-% bis 80 Gew.-%, vorzugsweise ungefähr 2 Gew.-%, enthält.

## Claims

1. Use of at least one protein fraction extracted from the seeds of a plant of the genus Moringa belonging to the Moringaceae family, as an active principle, alone or in combination with at least one other active principle, for the preparation of a cosmetic and/or pharmaceutical composition for topical use on the skin, lips and/or superficial body growths.

2. Use according to claim 1, **characterised in that** the protein fraction(s) consist(s) of at least one or more extracts of the plant Moringa oleifera.

3. Use according to claims 1 and 2, **characterised in that** the protein fraction(s) is (are) extracted by water or an aqueous solution, particularly saline solutions with different pH values, if necessary by means of an ultrasonic generator.

4. Use according to any one of claims 1 to 3, **characterised in that** the protein fraction(s) is (are) constituted by one or more aqueous extract(s) or in a buffered medium of whole or hulled seeds with lipids partly or totally removed, by a protein concentrate, by purified proteins or by a mixture of at least two of the above-mentioned constituents.

5. Use according to any one of claims 1 to 4, **characterised in that** the protein fraction(s) contain(s), based on the dry extract, a protein content of between 0.01% and 100% by weight, preferably about 45% by weight.

6. Use according to any one of claims 1 to 5, **characterised in that** the protein fraction(s) comprise(s) at least one protein compound whose isoelectric point is above 7, preferably between 8 and 12.

7. Use according to any one of claims 1 to 6, **characterised in that** the protein fraction(s) is (are) obtained by precipitation at the isoelectric point at a pH of between 8 and 12.

8. Use according to any one of claims 1 to 6, **characterised in that** the protein fraction(s) is (are) obtained by ion exchange chromatography.

9. Use according to any one of claims 1 to 6, **characterised in that** the protein fraction(s) is (are) obtained by a preparation process chosen from the group made up of affinity chromatography, gel filtration, ultrafiltration, precipitation using solvents, salts such as ammonium sulphate or the like, or precipitation using organic polymers or temperature variations.

10. Use according to any one of claims 1 to 9, **characterised in that** the protein fraction(s) consist(s) of native proteins.

11. Use according to any one of claims 1 to 9, **characterised in that** the protein fraction(s) consist(s) of a chemical or enzymatic hydrolyzate prepared from native proteins.

12. Use according to any one of claims 1 to 9, **characterised in that** the protein fraction(s) is (are) obtained by polymerisation of native proteins extracted from the plant of the genus Moringa or fermentation by microbial or vegetable cells.

13. Use according to any one of claims 1 to 9, **characterised in that** the protein fraction(s) are chemically modified by grafting of compounds such as, for example, oses, osides, lipids or similar.

14. Use according to any one of claims 1 to 13, **characterised in that** the protein fractions are incorporated or combined with a suitable cosmetic vector such as, for example, film forming agents, liposomes, cyclodextrins, micella, chylomicrons, macro-, micro- or nano-particles, or macro-, micro- or nano-capsules.

15. Use according to any one of claims 1 to 13, **characterised in that** the protein fractions are absorbed or grafted on organic polymers or mineral supports.

16. Use according to any one of claims 1 to 13, **characterised in that** the protein fractions extracted have substantive and hydrating actions on the skin, lips and superficial body growths, particularly the epidermis, and more particularly the stratum corneum and the cutaneous appendages, namely skin, hair and nails.

17. Use according to any one of claims 1 to 13, **characterised in that** the protein fractions extracted have physiological conditioning effects on the skin, lips and superficial body growths by restructuring, repairing and hydrating the skin, lips and superficial body growths, and also have anti-wrinkle and anti-pollution effects.

18. Cosmetic and/or pharmaceutical composition, particularly for topical use on the skin or superficial body growths, **characterised in that** it contains, by way of active principle, either alone or combined with at least one other active principle, one or more protein fraction(s) according to any one of claims 1 to 17, with a content by weight of between 0.01% and 80%, preferably about 2%.
